# EUROPÄISCHE PATENTSCHRIFT

(11) **EP 1 838 650 B1**
(45) Veröffentlichungstag und Bekanntmachung des Hinweises auf die Patenterteilung: **08.09.2010**
(21) Anmeldenummer: 05843706.2
(22) Anmeldetag: 17.12.2005
(51) Int. Cl.: C07C 29/56, C07C 35/17

(54) **VERFAHREN ZUR HERSTELLUNG VON ISOPULEGOL**
METHOD FOR THE PRODUCTION OF ISOPULEGOL
PROCEDE DE PRODUCTION D'ISOPULEGOL

(30) Priorität: 22.12.2004 DE 102004063003
(43) Veröffentlichungstag der Anmeldung: 03.10.2007
(73) Patentinhaber: BASF SE, 67056 Ludwigshafen (DE)
(72) Erfinder: FRIEDRICH, Marko, 64653 Lorsch (DE); EBEL, Klaus, 68623 Lampertheim (DE); GÖTZ, Norbert, 67547 Worms (DE)
(86) Internationale Anmeldenummer: PCT/EP2005/013628
(87) Internationale Veröffentlichungsnummer: WO 2006/069659

(56) Entgegenhaltungen:
- EP-A- 0 926 117
- EP-A- 1 225 163
- WO-A-2004/092099
- WO-A-2004/101480

## Beschreibung

### Technisches Gebiet der Erfindung:

Die Erfindung betrifft ein Verfahren zur Herstellung von Isopulegol bzw. Isomerengemischen des Isopulegols durch Cyclisierung von Citronellal in Gegenwart von Tris(aryloxy)aluminium-Katalysatoren. Insbesondere betrifft die Erfindung ein Verfahren zur Herstellung von optisch aktivem Isopulegol bzw. dessen Isomerengemische durch Cyclisierung von optisch aktivem Citronellal.

Menthol stellt weltweit die mengenmäßig wichtigste Aromachemikalie dar. Der Bedarf an Menthol wird nach wie vor zu einem großen Teil durch Isolierung aus natürlichen Quellen gedeckt. Daneben existieren jedoch auch synthetische Zugänge zum Menthol, teils in racemischer Form, teils in Form des natürlichen Enantiomeren L-Menthol.

Ein wichtiges Zwischenprodukt zur Herstellung von optisch aktivem Menthol stellt Isopulegol dar, das üblicherweise durch cyclisierende Oxo-En-Reaktion von Citronellal in Gegenwart von Lewis-sauren Katalysatoren hergestellt wird und üblicherweise in Form von Gemischen der vier Diastereomere Isopulegol, iso-Isopulegol, neo-Isopulegol und neoiso-Isopulegol anfällt.

### Stand der Technik:

Die EP-A1, 225,163 beschreibt ein Verfahren zur Herstellung von Isopulegol durch selektive Cyclisierung von Citronellal in Gegenwart spezieller Tris(2,6-diarylaryloxy)aluminium-Katalysatoren, insbesondere des Tris(2,6-diphenylphenol)aluminium-Komplexes. Bemerkenswert ist dabei vor allem die hohe Selektivität des Katalysators bezüglich der Bildung des gewünschten Diastereomeren (Isopulegol).

Bei Durchführung der Cyclisierungsreaktion in Gegenwart der in der EP-A 1,225,163 beschriebenen Katalysatoren wurde nun beobachtet, dass dabei eine unerwünschte Nebenreaktion in störendem Ausmaß stattfindet: Dabei handelt es sich um die Bildung von Citronellsäurecitronellylester der Formel (XII) 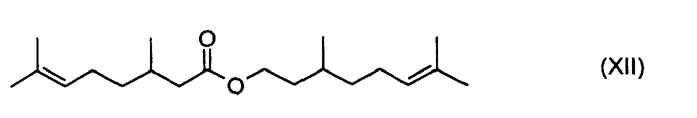 oder anderer hochsiedender Verunreinigungen, was bei den an die vorstehend genannte Reaktion zu stellenden hohen Anforderungen an Ausbeute und Selektivität nicht hingenommen werden kann.

### Aufgabe der Erfindung:

Aufgabe der vorliegenden Eindung war es, ein Verfahren bereitzustellen, dass es ermöglicht, die Cyclisierung von Citronellal zu Ispulegol in Gegenwart von tris(2,6-Diarylaryloxy)aluminium-Katalysatoren so durchzuführen, dass dabei die Bildung höhersiedender Reaktionsprodukte weitgehend unterdrückt wird.

Beschreibung der Erfindung sowie der bevorzugten Ausführungsformen:

Die Aufgabe wurde erfindungsgemäß gelöst durch die Bereitstellung eines Verfahrens zur Herstellung von Isopulegol der Formel (I) 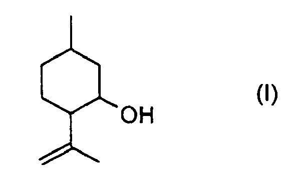 umfassend die Cyclisierung von Citronellal der Formel (II) 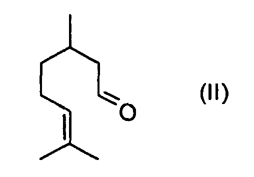 in Gegenwart eines tris(Aryloxy)aluminium-Katalysators der Formel (III) 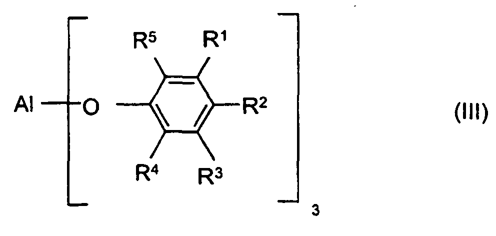 wobei
- Al: Aluminium bedeutet und
- R¹, R², R³: jeweils unabhängig voneinander Wasserstoff, ein Halogenatom, einen Alkylrest mit 1 bis 8 Kohlenstoffatomen, eine Alkoxygruppe mit 1 bis 8 Kohlenstoffatomen, einen substituierten oder unsubstituierten Arylrest, eine Dialkylaminogruppe wobei jeder Alkylrest 1 bis 4 Kohlenstoffato- me aufweisen kann, oder eine Nitrogruppe und
- R⁴, R⁵: jeweils unabhängig voneinander ein Halogenatom, einen Alkylrest mit 1 bis 8 Kohlenstoffatomen, eine Alkoxygruppe mit 1 bis 8 Kohlenstoffato- men, eine Dialkylaminogruppe wobei jeder Alkylrest 1 bis 4 Kohlenstoff- atome aufweisen kann, eine Nitrogruppe oder einen substituierten oder unsubstituierten Aryl- oder Heteroarylrest bedeuten können,
das dadurch gekennzeichnet ist, dass man die Cyclisierung in Gegenwart
I. mindestens einer Säure und/oder
II. mindestens einer Verbindung, ausgewählt aus der Gruppe der Carbonsäureanhydride, Vinylether, Ketone und Aldehyde der Formel (VII) 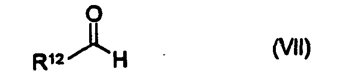
wobei
- R¹²: einen verzweigten oder unverzweigten C₁- bis C₁₂- Alkylrest oder C₇- bis C₁₂-Aralkylrest oder einen C₆- bis C₁₀-Arylrest bedeutet, wobei die genannten Reste jeweils einen oder mehrere gleiche oder verschiedene Substituenten, ausgewählt aus der Gruppe OR⁷, SR⁸ NR⁹R¹⁰ und Halogen aufweisen können, wobei R⁷, R⁸, R⁹ und R¹⁰ unabhängig voneinander C₁- bis C₆-Alkyl, C₇- bis C₁₂- Aralkyl und/oder substituiertes oder unsubstituiertes C₆- bis C₁₀- Aryl bedeuten können,
durchführt.

Das erfindungsgemäße Verfahren zeichnet sich dadurch aus, dass man die durch einen Tris(aryloxy)aluminium-Komplex der Formel (III) katalysierte Cyclisierung von Citronellal zu Isopulegol in Gegenwart mindestens einer Säure und/oder in Gegenwart mindestens einer Verbindung, die ausgewählt ist aus der Gruppe der Carbonsäureanhydride, Aldehyde, Ketone und Vinylether, durchführt. Bevorzugt führt man die Cylisierung in Gegenwart mindestens einer Verbindung, ausgewählt aus der Gruppe der Carbonsäureanhydride, Aldehyde, Ketone und Vinylether, bevorzugt ausgewählt aus der Gruppe der Carbonsäureanhydride, Aldehyde und Ketone durch.

Das erfindungsgemäße Verfahren eignet sich auch zur Herstellung von Isopulegol in optisch aktiver Form durch Cyclisierung von optisch aktivem Citronellal. Dabei können wahlweise beide Enantiomere des Citronellals in erfindungsgemäßer Weise eingesetzt werden. Vorzugsweise setzt man Citronellal ein das einen Enantiomerenüberschuss von etwa 90 bis etwa 100%ee, bevorzugt etwa 95 bis etwa 99% ee aufweist. Besonders bevorzugt setzt man als Ausgangsstoff zur Durchführung des erfindungsgemäßen Verfahrens D-Citronellal der Formel (XI) 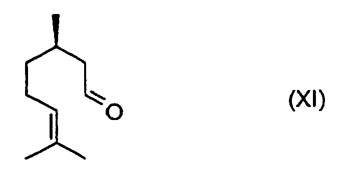 ein und erhält daraus L-Isopulegol der Formel (X)

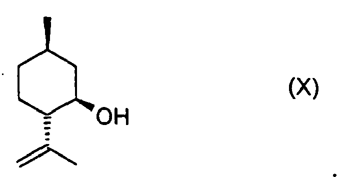

Unabhängig davon, in welcher Reinheit bzw. in Form welches Enantiomeren Citronellal eingesetzt wird, erhält man Isopulegol in der Regel in Form von Gemischen der vier Diastereomere Isopulegol, iso-Isopulegol, neo-Isopulegol und neo-iso-Isopulegol.

Das erfindungsgemäße Cyclisierungsverfahren zur Herstellung von Isopulegol kann in Gegenwart einer oder verschiedener Säuren, bevorzugt in Gegenwart einer Carbonsäure mit 1 bis 20 Kohlenstoffatomen durchgeführt werden. Als geeignete Carbonsäuren sind beispielsweise Ameisensäure Essigsäure, Propionsäure, Buttersäure, Valeriansäure, Oxalsäure, Malonsäure, Bernsteinsäure und Citronellsäure, insbesondere Essigsäure bevorzugt. Die genannten Carbonsäuren mit 1 bis 20 Kohlenstoffatomen können auch in Form von Gemischen untereinander im Rahmen des erfindungsgemäßen Verfahrens eingesetzt werden.

In einer weiteren Ausführungsform lässt sich das erfindungsgemäße Cyclisierungsverfahren vorteilhaft auch in Gegenwart von anorganischen Säuren durchführen. Geeignete anorganische Säuren sind beispielsweise HCl (Salzsäure), die als wässrige Lösung oder als Lösung in organischen Lösungsmitteln oder auch gasförmig eingesetzt werden kann, Schwefelsäure, Salpetersäure oder Phosphorsäure oder Gemische der genannten Säuren.

Die genannten Säuren werden dem erfindungsgemäß umzusetzendem Citronellal üblicherweise in Mengen von etwa 0,01 bis etwa 5 Gew.-%, bevorzugt von etwa 0,1 bis etwa 2,5 Gew.-% zugegeben.

Das erfindungsgemäße Verfahren zur Herstellung von Isopulegol durch Cyclisierung von Citronellal lässt sich in einer bevorzugten Ausführungsform erfindungsgemäß auch in Gegenwart mindestens einer Verbindung, ausgewählt aus der Gruppe der Carbonsäureanhydride, Aldehyde, Ketone und Vinylether durchführen.

Die Verbindungen der genannten Substanzklassen können jeweils einzeln oder in Form von Gemischen untereinander eingesetzt werden. Bevorzugt setzt man im Fall von Gemischen solche ein, die aus Verbindungen einer Substanzklasse bestehen. Besonders bevorzugt setzt man einzelne Verbindungen ein.

Bevorzugt führt man die erfindungsgemäße Cyclisierung in Gegenwart eines Carbonsäureanhydrids der Formel (VI) 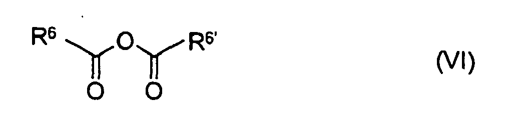 durch, wobei die Reste R⁶ und R^{6'} gleich oder verschieden, bevorzugt gleich, sein können und einen verzweigten oder unverzweigten C₁- bis C₁₂- Alkylrest oder C₇- bis C₁₂-Aralkylrest oder einen C₆- bis C₁₀-Arylrest bedeuten, wobei die genannten Reste jeweils einen oder mehrere, in der Regel 1 bis etwa 3, gleiche oder verschiedene Substituenten, ausgewählt aus der Gruppe OR⁷, SR⁸ NR⁹R¹⁰ und Halogen aufweisen können und wobei R⁶ und R^{6'} gemeinsam auch einen 5- bis 8-gliedrigen Ring bilden können, der eine oder mehrere, bevorzugt 1 oder 2, ethylenische Doppelbindungen und ein oder mehrere, bevorzugt 1 oder 2 gleiche oder verschiedene Heteroatome, ausgewählt aus der Gruppe O, S, NR¹¹ aufweisen kann und wobei R⁷, R⁸, R⁹, R¹⁰ und R¹¹ jeweils unabhängig voneienander C₁- bis C₆-Alkyl, C₇- bis C₁₂-Aralkyl und/oder substituiertes oder unsubstituiertes C₆- bis C₁₀-Aryl bedeuten können.

Dabei seien für die genannten Reste R⁷ bis R¹¹ beispielhaft die folgenden Bedeutungen angegeben: C₁-C₆-Alkyl wie z.B. Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl; C₇- bis C₁₂-Aralkyl wie z.B. Benzyl, 1-Phenylethyl, 2-Phenylethyl; C₆- bis C₁₀-Aryl wie Phenyl oder Naphthyl.

Ebenfalls bevorzugt führt man das erfindungsgemäße Verfahren in Gegenwart eines Aldehyds der Formel (VII) 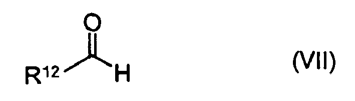 durch, wobei der Rest R¹² einen verzweigten oder unverzweigten C₁- bis C₁₂- Alkylrest oder C₇- bis C₁₂-Aralkylrest oder einen C₆- bis C₁₀-Arylrest bedeutet, wobei die genannten Reste jeweils einen oder mehrere, bevorzugt 1 bis 3, gleiche oder verschiedene Substituenten, ausgewählt aus der Gruppe OR⁷, SR⁸ N⁹R¹⁰ und Halogen aufweisen können, wobei die Reste R⁷ bis R¹⁰ die vorstehend angegebenen Bedeutungen haben können.

Ebenfalls bevorzugt führt man das erfindungsgemäße Verfahren in Gegenwart eines Ketons der Formel (VIII) 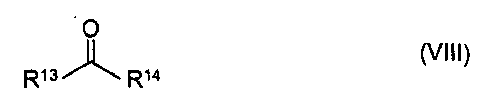 durch, wobei die Reste R¹³ und R¹⁴ jeweils gleich oder verschieden sein können und einen verzweigten oder unverzweigten C₁- bis C₁₂- Alkylrest oder C₇- bis C₁₂-Aralkylrest oder einen C₆- bis C₁₀-Arylrest bedeuten, wobei die genannten Reste jeweils einen oder mehrere, bevorzugt 1 bis 3, gleiche oder verschiedene Substituenten, ausgewählt aus der Gruppe OR⁷, SR⁸ N⁹R¹⁰ und Halogen aufweisen können, und wobei R¹³ und R¹⁴ gemeinsam auch einen 5- bis 8-gliedrigen Ring bilden können, der eine oder mehrere, bevorzugt 1 oder 2, ethylenische Doppelbindungen und ein oder mehrere, bevorzugt 1 oder 2, gleiche oder verschiedene Heteroatome ausgewählt aus der Gruppe O, S, NR¹¹ aufweisen kann und wobei die Reste R⁷ bis R¹¹ die vorstehend angegebenen Bedeutungen haben können.

Alternativ zu den genannten Carbonylverbindungen lassen sich auch Vinylether bzw. Vinylester der allgemeinenen Formel (IX) 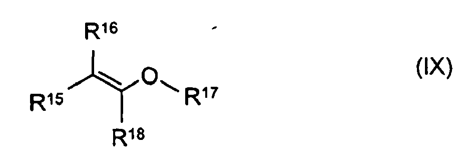 im Rahmen des erfindungsgemäßen Verfahrens einsetzen, wobei die Reste R¹⁵, R¹⁶, R¹⁷ und R¹⁸ unabhängig voneinander jeweils gleich oder verschieden sein können und einen verzweigten oder unverzweigten C₁- bis C₁₂- Alkylrest oder C₇- bis C₁₂-Aralkylrest oder einen C₆- bis C₁₀-Arylrest bedeuten, wobei die genannten Reste jeweils einen oder mehrere, bevorzugt 1 bis 3, gleiche oder verschiedene Substituenten, ausgewählt aus der Gruppe Oxo, OR⁷, SR⁸ N⁹R¹⁰ und Halogen aufweisen können und wobei R¹⁶ und R¹⁷ gemeinsam auch einen 5- bis 8-gliedrigen Ring bilden können, der eine oder mehrere, bevorzugt 1 oder 2, ethylenische Doppelbindungen und ein oder mehrere, üblicherweise 1 oder 2, gleiche oder verschiedene Heteroatome ausgewählt aus der Gruppe O, S, NR¹¹ aufweisen kann und wobei die Reste R⁷ bis R¹¹ die vorstehend angegebenen Bedeutungen haben können.

C₁- bis C₁₂- Alkyl steht dabei für wie vorstehend beschriebenes C₁- bis C₆-Alkyl und darüber hinaus beispielsweise für Heptyl, Octyl, Nonyl, Decyl, Undecyl oder Dodecyl. In den Fällen, in denen zwei Alkylreste gemeinsam einen Ring ausbilden, sind unter Alkylresten auch Alkylenylreste zu verstehen. C₇- bis C₁₂-Aralkylresten und C₆- bis C₁₀-Arylresten können beispielhaft die vorstehend genannten Bedeutungen zukommen.

Im Rahmen einer bevorzugten Ausführungsform führt man das erfindungsgemäße Verfahren in Gegenwart eines Carbonsäureanhydrids der Formel (VI) durch, wobei die Reste R⁶ und R^{6'} gleich sind und einen verzweigten oder unverzweigten C₁- bis C₁₂-Alkylrest oder C₇- bis C₁₂-Aralkylrest oder einen C₆- bis C₁₀-Arylrest bedeuten, und wobei R⁶ und R^{6'} gemeinsam auch einen 5- bis 8-gliedrigen Ring bilden können, der eine oder mehrere, bevorzugt eine oder 2 ethylenische Doppelbindungen und ein oder mehrere, bevorzugt 1 oder 2 gleiche oder verschiedene Heteroatome ausgewählt aus der Gruppe O, S, NR¹¹ aufweisen kann und wobei die Reste R⁷ bis R¹¹ die vorstehend angegebenen Bedeutungen haben können.

Insbesondere bevorzugt setzt man solche Carbonsäureanhydride ein, bei denen die Reste R⁶ und R^{6'} gleich sind und einen verzweigten oder unverzweigten C₁- bis C₁₂-Alkylrest oder einen C₆- bis C₁₀-Arylrest bedeuten. Beispielhaft seien als erfindungsgemäß besonders bevorzugt einzusetzende Carbonsäureanhydride genannt: Essigsäureanhydrid, Propionsäureanhydrid, Pivalinsäureanhydrid und Benzoesäureanhydrid.

Als erfindungsgemäß ebenfalls bevorzugt einsetzbare Aldehyde der Formel (VII) seien bespielhaft Acetaldehyd, Propionaldehyd und Chloral genannt.

Führt man das erfindungsgemäße Cyclisierungsverfahren im Rahmen einer weiteren bevorzugten Ausführungsform in Gegenwart eines Ketons der Formel (VIII) durch, setzt man mit Vorteil solche mit einer aktivierten, d.h. elektronenarmen Carbonylfunktion ein. Beispielhaft seien die folgenden Ketone genannten, die sich in besonderem Maße zum Einsatz im Rahmen des erfindungsgemäßen Verfahren eigenen: 1,1,1-Trifluoraceton, 1,1,1-Trifluoracetophenon und Hexafluoraceton.

Als erfindungsgemäß ebenfalls bevorzugt einsetzbare Vinylether der Formel (IX) seien beispielhaft genannt: Methylvinylether, Ethylvinylether, Isobutylvinylether und 3,4-Dihydro-2H-pyran, 2-Methoxypropen. Daneben eignen sich auch die entsprechenden Vinylester zu Einsatz im Rahmen des erfindungsgemäßen Verfahrens.

Die genannten Verbindungsklassen können gleichermaßen mit gutem Erfolg im Rahmen dieser Ausführungsform des erfindungsgemäßen Verfahrens eingesetzt werden. Im Hinblick auf praktische Gesichtspunkte wie beispielsweise eine höhere Reaktionsgeschwindigkeit hat sich der Einsatz von Aldehyden der Formel (VII) und/oder elektronenarmen Ketonen der Formel (VIII) als vorteilhaft erweisen.

Die Menge an erfindungsgemäß im Rahmen dieser Ausführungsform einzusetzendem Carbonsäureanhydrid, Aldehyd, Keton und/oder Vinylether kann in breiten Grenzen variiert werden und richtet sich nach Art des eingesetzten Stoffes und dem Reinheitsgrad bzw. dem Vorhandensein noch nicht näher identifizierter Verunreinigungen. Üblicherweise setzt man die genannten Verbindungen bzw. deren Gemische in einer Menge von 0,01 mol-% bis 5 mol-%, bevorzugt von 0,1 mol-% bis 2 mol-% bezogen auf die eingesetzte Menge an Citronellal ein.

An die Art und Weise der Reaktionsführung, beispielsweise die Ausgestaltung von Reaktoren oder die Reihenfolge der Zugabe einzelner Reaktionspartner sind keine besonderen Anforderungen zu stellen, solange eine Reaktionsführung unter weitgehendem Ausschluss von Sauerstoff und Wasser gewährleistet ist.

Zur Durchführung des erfindungsgemäßen Verfahrens im Rahmen dieser Ausführungsform geht man vorteilhaft so vor, dass man zunächst eine Lösung der erfindungsgemäß einzusetzenden Tris(aryloxy)aluminium-Katalysators der Formel (III) bzw. (IV) in einem geeigneten Lösungsmittel wie nachstehend beschrieben bereitstellt. Dieser Lösung setzt man dann erfindungsgemäß bevorzugt ein Gemisch des zu cyclisierenden racemischen oder nicht-racemischen Citronellals mit dem gewählten Carbonsäureanhydrid, dem Aldehyd, dem aktivierten Keton und/oder dem Vinylether zugibt. Alternativ dazu kann man beispielsweise auch die Lösung der erfindungsgemäß einzusetzenden Tris(aryloxy)aluminium-Katalysators der Formel (III) bzw. (IV) zunächst mit dem Carbonsäureanhydrid, dem Aldehyd, dem Keton und/oder dem Vinylether versetzen und im Anschluss daran das zu cyclisierende Citronellal zugeben.

Es sich als vorteilhaft erwiesen, das Citronellal bzw. das Gemisch von Citronellal mit der gewählten Verbindung innerhalb eines Zeitraums von 30 min bis 6 h, bevorzugt innerhalb von 2 h bis 3 h zur Katalysatorlösung bzw. dem Reaktionsgemisch zuzudosieren. Das Citronellal kann dabei als solches oder in Form einer Lösung, vorteilhaft in einem der vorstehend genannten geeigneten Lösungsmittel zugegeben werden. Im Rahmen einer bevorzugten Ausführungsform des erfindungsgemäßen Verfahrens bereitet man zunächst eine Lösung des dem gewählten Katalysator entsprechenden Aryloxy-Liganden in einem geeigneten Lösungsmittel wie beispielsweise in Toluol und setzt dann, zweckmäßigerweise unter Rühren, eine geeignete Aluminium-Verbindung wie beispielsweiseTrimethyl- oder Triethylaluminium in toluolischer Lösung zu.

Die Zugabe des zu cyclisierenden Citronellals bzw. des Gemischs von Cironellal mit dem gewählten Carbonsäureanhydrid, Aldehyd, aktivierten Keton und/oder dem Vinylether erfolgt vorteilhaft bei Temperaturen im Bereich von -40°C bis 40°C, bevorzugt im Bereich von -20°C bis 20°C. Dazu wird vorteilhaft die bereitete Lösung oder Suspension des erfindungsgemäß einzusetzenden Katalysators auf eine Temperatur in diesem Bereich, z.B. auf eine Temperatur im Bereich von -10°C bis 10°C abgekühlt und die weiteren Reaktanden in vorgekühlter Form zugegeben.

Die Zugabe des Gemischs von Citronellal und der gewählten weiteren Verbindung kann so vorgenommen werden, dass man entweder die gesamte Menge auf einmal zusetzt oder sie portionsweise oder auch kontinuierlich zur bereiteten Katalysatorlösung gibt. Als Lösungsmittel eignen sich wiederum die vorstehend genannten Lösungsmittel, insbesondere Toluol. Bevorzugt setzt man das zu cyclisierende Citronellal in Form eines Gemisches mit dem gewählten gewählten Carbonsäureanhydrid, Aldehyd, aktivierten Keton und/oder Vinylether ohne weiteren Zusatz von Lösungsmitteln ein. Bei Einsatz eines Lösungsmittels wird die gesamte Lösungsmittelmenge vorteilhaft so gewählt, dass das volumenbezogene Verhältnis von umzusetzendem Citronellal zum Lösungsmittel 1:1 bis 1:20, bevorzugt von 1:1 bis 1:10 beträgt.

Das erfindungsgemäße Verfahren wird in Gegenwart von Tris(aryloxy)aluminium-Katalysatoren der Formel (III) 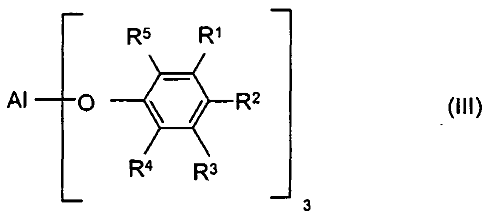 wobei
Al Aluminium bedeutet und
- R¹, R², R³: jeweils unabhängig voneinander Wasserstoff, ein Halogenatom, einen Al- kylrest mit 1 bis 8 Kohlenstoffatomen, eine Alkoxygruppe mit 1 bis 8 Koh- lenstoffatomen, einen substituierten oder unsubstituierten Arylrest, eine Di- alkylaminogruppe wobei jeder Alkylrest 1 bis 4 Kohlenstoffatome aufweisen kann, oder eine Nitrogruppe bedeuten können und
- R⁴, R⁵: jeweils unabhängig voneinander ein Halogenatom, einen Alkylrest mit 1 bis 8 Kohlenstoffatomen, eine Alkoxygruppe mit 1 bis 8 Kohlenstoffatomen, ei- ne Dialkylaminogruppe wobei jeder Alkylrest 1 bis 4 Kohlenstoffatome auf- weisen kann, eine Nitrogruppe oder einen substituierten oder unsubstituier- ten Aryl- oder Heteroarylrest bedeuten können.

Beispielhaft seien für die Reste R¹, R² und R³ folgende Bedeutungen genannt: Wasserstoff; ein Halogenatom wie beispielsweise Fluor, Chlor, Brom oder Iod; eine Alkylgruppe mit 1 bis 8 Kohlenstoffatomen wie Methyl, Ethyl, Propyl, Isopropyl, n-Butyl, Isobutyl, sec-Butyl, tert-Butyl, Pentyl, Hexyl, Heptyl, Octyl; eine Alkoxygruppe mit 1 bis 8 Kohlenstoffatomen wie Methoxy, Ethoxy, Propoxy, Isopropoxy, Butoxy, Isobutoxy, sec-Butoxy, tert-Butoxy, Pentoxy, Hexoxy, Heptoxy, Octoxy; eine Phenylgruppe, die 1 bis 5 Substituenten tragen kann wie z.B. eine Alkylgruppe mit 1 bis 4 Kohlenstoffatomen (z.B. Methyl, Ethyl, Propyl, Isopropyl, n-Butyl, iso-Butyl, sec-Butyl, tert-Butyl), eine Alkoxygruppe mit 1 bis 4 Kohlenstoffatomen (z.B. Methoxy, Ethoxy, Propoxy, Isopropoxy, Butoxy, Isobutoxy, sec-Butoxy, tert-Butoxy), ein Halogenatom (z.B. Fluor, Chlor, Brom oder Iod) und dergleichen mehr; eine Naphthylgruppe, die 1 bis 7 Substituenten tragen kann wie z.B. eine Alkylgruppe mit 1 bis 4 Kohlenstoffatomen (z.B. Methyl, Ethyl, Propyl, Isopropyl, n-Butyl, iso-Butyl, sec-Butyl, tert-Butyl), eine Alkoxygruppe mit 1 bis 4 Kohlenstoffatomen (z.B. Methoxy, Ethoxy, Propoxy, Isopropoxy, Butoxy, Isobutoxy, sec-Butoxy, tert-Butoxy), ein Halogenatom (z.B. Fluor, Chlor, Brom oder Iod) und dergleichen mehr; eine Dialkylaminogruppe, wobei jeder Alkylrest 1 bis 4 Kohlenstoffatome aufweisen kann wie z.B. Dimethylamino, Diethylamino, Dipropylamino, Diisopropylamino, Dibutylamino; und Nitro.

Für die Reste R⁴ und R⁵ seien beispielhaft die vorstehend für R¹, R² und R³, ausgenommen Wasserstoff, aufgeführten Reste genannt sowie als Heteroarylreste Furyl, Thienyl, Pyranyl, Benzofuryl, Isobenzofuryl, Benzothienyl, Indolyl, Isoindolyl, Carbazolyl, Pyridyl, Chinolyl, Isochinolyl oder Pyrazyl, die jeweils einen oder mehrere der vorstehend genannten Substituenten tragen können.

In einer bevorzugten Ausführungsform führt man das erfindungsgemäße Verfahren in Gegenwart von Tris(2,6-diarylaryloxy)aluminium-Katalysatoren der Formel (IV) 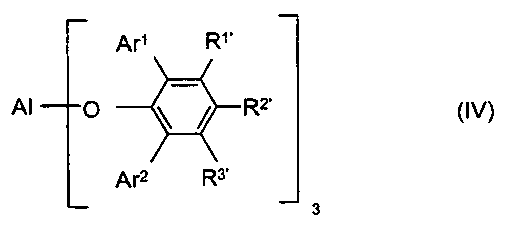 wobei
Al Aluminium bedeutet und
- Ar¹ und Ar²: jeweils unabhängig voneinander für einen substituierten oder unsubstitu- ierten Aryl- oder Heteroarylrest stehen und
- R^{1'}, R^{2'}, R^{3'}: jeweils unabhängig voneinander Wasserstoff, ein Halogenatom, einen Alkylrest mit 1 bis 8 Kohlenstoffatomen, eine Alkoxygruppe mit 1 bis 8 Kohlenstoffatomen, einen substituierten oder unsubstituierten Arylrest, eine Dialkylaminogruppe wobei jeder Alkylrest 1 bis 4 Kohlenstoffatome aufweisen kann, oder eine Nitrogruppe bedeuten können.

Derartige Katalysatoren und ihre Herstellung sind beispielsweise in der EP-A 1 225 163, auf die diesbezüglich hiermit Bezug genommen wird, beschrieben. Üblicherweise werden derartige Katalysatoren durch Umsetzung der entsprechenden 2,6-Disubstituierten Phenolliganden mit geeigneten Aluminiumverbindungen, beispielsweise Trimethylaluminium, Triethylaluminium oder auch Triisobutylaluminiumhydrid gewonnen.

Einen bevorzugten Katalysator stellt Tris(2,6-diphenyphenol)aluminium (Formel (IV) mit R^{1'}, R^{2'}, R^{3'} jeweils H und Ar¹ und Ar² jeweils Phenyl) der beispielsweise durch Umsetzung von 2,6-Diphenylphenol mit Triethylaluminium zugänglich ist. Weitere Katalysatoren sind in analoger Weise durch dem Fachmann bekannte Verfahren zugänglich.

Zur Durchführung des erfindungsgemäßen Verfahrens setzt man üblicherweise 0.1 bis 5 mol-%, bevorzugt 1 bis 3 mol-% (bezüglich der Menge an umzusetzendem Citronellal) eines der genannten Katalysatoren ein.

Der Katalysator wird normalerweise nach wie vorstehend beschriebener Vorbehandlung des Citronellals bzw. nach Zusatz der Oxidationsprodukte des Citronellals oder nach Zusatz der gewählten Säure oder der Verbindung, die ausgewählt aus der Gruppe der Carbonsäureanhydride, Aldehyde, Ketone und Vinylether, mit dem Edukt bzw. einer Lösung des Eduktes in Kontakt gebracht. Als geeignete Lösemittel seien Toluol, Ethylacetat, Diethylether, Methyl-tert.-butylether, Dichlormethan, Chloroform, Tetrachlormethan, Chlorbenzol, Isopropylacetat, Hexan, Heptan, Cyclohexan, THF, Aceton genannt. Es kann auch unter lösemittelfreien Bedingungen gearbeitet werden.

Die erfindungsgemäße Cyclisierung wird vorteilhaft bei Temperaturen von - 20 bis 40°C, bevorzugt bei 0 bis 10°C durchgeführt und ist in der Regel nach 1 bis 10 h, oft nach 1 bis 5 h abgeschlossen. Die Aufarbeitung des erhaltenen Reaktionsgemisches ist nicht kritisch und kann nach allen dem Fachmann geeignet erscheinenden Verfahren vorgenommen werden.

Durch das erfindungsgemäße Cyclisierungsverfahren erhält man Isopulegol, üblicherweise in Form von Gemischen der vier Diastereomere Isopulegol, iso-Isopulegol, neo-Isopulegol und neoiso-Isopulegol, wobei der überwiegende Teil, in der Regel weit über 80%, oft deutlich über 90% des Gemisches aus dem gewünschten Diastereomeren Isopulegol besteht.

Die erfindungsgemäß erhaltenen Produkte bzw. Produkt-Gemische zeichnen sich durch einen geringen Gehalt bzw. durch weitgehende bis vollsändige Abwesenheit an unerwünschten höhersiedenden Nebenprodukten wie z.B. den vorstehend genannte Citronellsäurecitronellylester der Formel (XII) 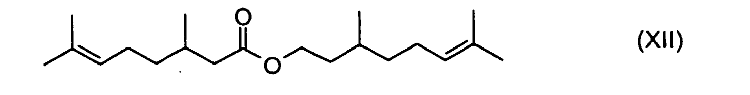 aus. Je nach Art der Vorbehandlungsbedingungen zur Erzeugung der genannten Oxidationsprodukte bzw. je nach Art oder Menge der eingesetzten Oxidationsprodukte oder Carbonsäuren kann die Bildung von Citronellsäurecitronellylester der Formel (XII) weitestgehend unterdrückt werden, oft auf einen Anteil von 0 bis 5 Gew.-% bezogen auf die Gesamtmenge des Isopulegol enthaltenden Produktgemisches.

Das dargestellte Verfahren zur Herstellung von Isopulegol durch Cyclisierung von Citronellal in Gegenwart von tris(2,6-Diarylaryloxy)aluminium-Katalysatoren und Oxidationsprodukten des Citronellals bzw. organischen Säuren oder den weiteren genannten Verbindungen eröffnet somit einen ökonomisch besonders vorteilhaften Zugang zum Isopulegol ausgehend von in technischem Maßstab hergestelltem Citronellal. Durch das Verfahren gelingt es mit einfach zu realiserenden Mitteln, beispielsweise ohnehin durchzuführenden Destillation der Ausgangsstoffe unter speziellen Bedingungen, die Bildung unerwünschter, schwer abtrennbarer Nebenprodukte weitgehend zu unterdrücken.

Das gemäß dem erfindungsgemäßen Verfahren herstellbare racemische bzw. nichtracemische Isopulegol stellt ein wertvolles Intermediat zur Herstellung von racemischem bzw. nicht-racemischem Menthol, einem der weltweit bedeutensten Riech- bzw. Aromastoffe dar. Menthol kann durch dem Fachmann an sich bekannte Methoden der Hydrierung, speziell der katalytischen Hydrierung an geeigneten Übergangsmetallkatalysatoren, wie beispielsweise in Pickard et al., J. Chem. Soc. 1920, 1253; Ohloff et al., Chem. Ber. 1962, 95, 1400; Pavia et al., Bull. Soc. Chim. Fr. 1981, 24, Otsuka et al., Synthesis 1991, 665 oder in der EP 1 053 974 A beschrieben, aus Isopulegol erhalten werden. Dabei bleibt bei geeigneter Wahl der Reaktionsbedingungen die relative bzw. absolute Konfiguration des eingesetzten Isopulegols weitgehend, in vielen Fällen vollständig erhalten.

Die vorliegende Erfindung betrifft daher auch ein Verfahren zur Herstellung von racemischem oder nicht-racemischem Menthol, umfassend die Herstellung von Isopulegol der Formel (I) bzw. (X) durch Cyclisierung von racemischem oder nicht-racemischem Isolpulegol der Formel (II) bzw. (XI) nach dem vorstehend beschriebenen Verfahren und anschließende Hydrierung, bevorzugt katalytischen Hydrierung der ethylenischen Doppelbindung des so hergestellten Isopulegols. Insbesondere betrifft die vorliegende Erfindung ein Verfahren zur Herstellung von L-(-)-Menthol ausgehend von nach dem vorstehend beschriebenen Verfahren durch erfindungsgemäße Cyclisierung von D-(+)-Citronellal hergestelltem L-(-)-Isopulegol.

### Beispiele:

Die folgenden Beispiele dienen der Erläuterung der Erfindung, ohne sie in irgendeiner Weise einzuschränken:

### Beispiel 1: (vergleich)

### Allgemeine Versuchsvorschrift:

504 mg (2,05 mmol) 2,6-Diphenylphenol und 10 ml wasserfreies Toluol wurden vorgelegt und bei Raumtemperatur 350 µl (0,66 mmol) einer 0,66 molaren Lösung Triethylaluminium zugegeben. Die Lösung wurde 1 h bei 25°C gerührt, anschließend auf 0°C abgekühlt und 10,15 g (65,8 mmol) auf -15°C vorgekühltes Citronellal zugetropft. Das Reaktionsgemisch wurde 3 h bei 0°C gerührt und anschließend mit 8 ml 8%-iger Natronlauge versetzt. Aus der organischen Phase des erhaltenen zweiphasigen Gemisches wurde eine Probe entnommen und wie folgt beschrieben gaschromatographisch analysiert. Alle im Folgenden angegebenen Messwerte sind GC-Flächen-%-Werte.

Man erhielt ein Gemisch aus 4,65% Citronellal, 0,06% neo-Isopulegol, 31,74% Isopulegol, 0,12% neoiso-Isopulegoi, 0,04% iso-Isopulegol und 37,32% Citronellsäurecitronellylester.

### GC-Analysemethode:

Stationäre Phase: 30 m DB-WAX, Innendurchmesser: 0,32 mm; Flammenionisationsdetektor, Temperatur: 80°C - 230°C; Heizrate 3°C/min; Retentionszeiten: Rf (Citronellal): 10,5; Rf (neo-Isopulegol): 13,24; Rf (Isopulegol): 13,58, Rf (neoiso-Isopulegol): 14,64; Rf (iso-Isopulegol): 15,28; Rf (Cironellsäurecitronellylester): 39,80.

### Beispiele 2 bis 4:

Gemäß Beispiel 1 wurde Citronellal umgesetzt, das zuvor mit der in Tabelle 1, Spalte 2 angegebenen Menge Citronellsäure versetzt wurde. Die Ergebnisse sind in Tabelle 1 zusammengestellt.

**Tabelle 1:**

| Beispiel | Zugabe Citronellsäure | Citronellal | Neo-Isopulegol | Iso-pulegol | Neoiso-Isopulegol | Iso-Isopulegol | Ester* |
|---|---|---|---|---|---|---|---|
| 2 | keine | 33,41 | 0,09 | 39,39 | 0,36 | 0,05 | 10,19 |
| 3 | 0,1 Gew.-% | 31,81 | 0,10 | 44,02 | 0,33 | 0,06 | 7,89 |
| 4 | 0,5 Gew.-% | 60,79 | 0,08 | 30,22 | 0,29 | 0,04 | 0,74 |

| | | | | | | | |
|---|---|---|---|---|---|---|---|
| * Citronellsäurecitronellylester | | | | | | | |

### Beispiele 5 bis 8:

Gemäß Beispiel 1 wurde Citronellal umgesetzt, das zuvor mit der in Tabelle 2, Spalte 2 angegebenen Menge Essigsäure versetzt wurde. Die Ergebnisse sind in Tabelle 2 zusammengestellt.

**Tabelle 2:**

| Beispiel | Zugabe Essigsäure | Citronellal | Neo-Isopulegol | Isopulegol | Neoiso-Isopulegol | Iso-Isopule-gol | Ester* |
|---|---|---|---|---|---|---|---|
| 5 | keine | 2,67 | -- | 6,40 | 0,50 | - | 75,65 |
| 6 | 0,1 Gew.-% | 10,77 | 0,04 | 19,36 | 0,40 | 0,03 | 54,58 |
| 7 | 0,3 Gew.-% | 35,91 | 0,12 | 56,38 | 0,31 | 0,08 | 0,68 |
| 8 | 0,5 Gew.-% | 57,40 | 0,09 | 37,09 | 0,13 | 0,05 | 0,33 |

| | | | | | | | |
|---|---|---|---|---|---|---|---|
| * Citronellsäurecitronellylester | | | | | | | |

### Beispiel 9:

Gemäß Beispiel 1 wurde Citronellal umgesetzt, welches zuvor mit 1,0 Gew.-% Essigsäureanhydrid versetzt worden war. Die Reaktionsdauer betrug insgesamt 24 h. Das Ergebnis ist in Tabelle 3 zusammengestellt:

**Tabelle 3:**

| Zeit in h | Citronellal | Neo-Isopulegol | Isopulegol | Neoiso-Isopulegol | Iso-Isopulegol | Citronellol | Ester* |
|---|---|---|---|---|---|---|---|
| 1 | 10,40 | 0,11 | 52,28 | 0,20 | -- | -- | -- |
| 2 | 10,89 | 0,13 | 65,05 | 0,24 | 0,09 | -- | -- |
| 5 | 13,64 | 0,15 | 74,81 | 0,28 | 0,10 | -- | -- |
| 6 | 15,71 | 0,15 | 74,13 | 0,27 | 0,10 | -- | -- |
| 7 | 9,38 | 0,16 | 80,58 | 0,30 | 0,10 | -- | -- |
| 24 | 1,76 | 0,17 | 88,42 | 0,33 | 0,11 | -- | -- |

| | | | | | | | |
|---|---|---|---|---|---|---|---|
| * Citronellsäurecitronellylester | | | | | | | |

## Patentansprüche

1. Verfahren zur Herstellung von Isopulegol der Formel (I) 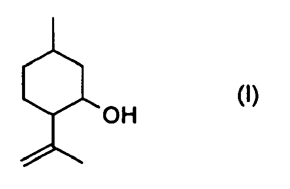 umfassend die Cyclisierung von Citronellal der Formel (II) 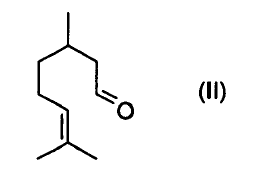 in Gegenwart eines Tris(aryloxy)aluminium-Katalysators der Formel (III) 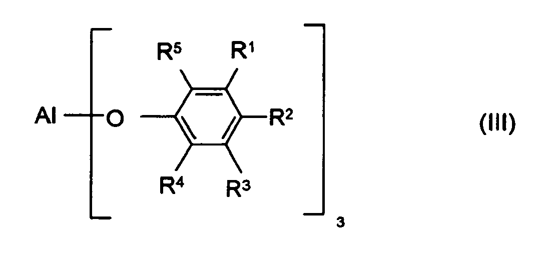 wobei
Al Aluminium bedeutet und
R¹, R², R³ jeweils unabhängig voneinander Wasserstoff, ein Halogenatom, einen Alkylrest mit 1 bis 8 Kohlenstoffatomen, eine Alkoxygruppe mit 1 bis 8 Kohlenstoffatomen, einen substituierten oder unsubsti- tuierten Arylrest, eine Dialkylaminogruppe wobei jeder Alkylrest 1 bis 4 Kohlenstoffatome aufweisen kann, oder eine Nitrogruppe und
R⁴, R⁵ jeweils unabhängig voneinander ein Halogenatom, einen Alkylrest mit 1 bis 8 Kohlenstoffatomen, eine Alkoxygruppe mit 1 bis 8 Koh- lenstoffatomen, eine Dialkylaminogruppe wobei jeder Alkylrest 1 bis 4 Kohlenstoffatome aufweisen kann, eine Nitrogruppe oder ei- nen substituierten oder unsubstituierten Aryl- oder Heteroarylrest bedeuten können,
**dadurch gekennzeichnet, dass** man die Cyclisierung in Gegenwart
I. mindestens einer Säure und/oder
II. mindestens einer Verbindung, ausgewählt aus der Gruppe der Carbonsäureanhydride, Vinylether, Ketone und Aldehyde der Formel (VII) 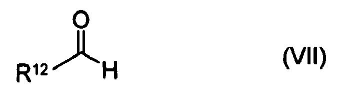 wobei
R¹² einen verzweigten oder unverzweigten C₁- bis C₁₂- Alkylrest oder C₇- bis C₁₂-Aralkylrest oder einen C₆- bis C₁₀-Arylrest bedeutet, wobei die genannten Reste jeweils einen oder mehrere gleiche oder verschiedene Substituenten, ausgewählt aus der Gruppe OR⁷, SR⁸ NR⁹R¹⁰ und Halogen aufweisen können, wobei R⁷, R⁸, R⁹ und R¹⁰ unabhängig voneinander C₁- bis C₆-Alkyl, C₇- bis C₁₂- Aralkyl und/oder substituiertes oder unsubstituiertes C₆- bis C₁₀- Aryl bedeuten können,
durchführt.

2. Verfahren nach Anspruch 1, **dadurch gekennzeichnet, dass** man als Tris(aryloxy)aluminium-Katalysator einen Tris(2,6-diarylaryloxy)aluminium-Katalysators der Formel (IV) 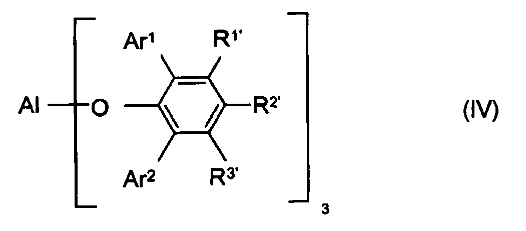 wobei
Al Aluminium bedeutet und
Ar¹, Ar² jeweils unabhängig voneinander für einen substituierten oder un- substituierten Aryl- oder Heteroarylrest stehen und
R^{1'}, R^{2'}, R^{3'} jeweils unabhängig voneinander Wasserstoff, ein Halogenatom, einen Alkylrest mit 1 bis 8 Kohlenstoffatomen, eine Alkoxygruppe mit 1 bis 8 Kohlenstoffatomen, einen substituierten oder unsubsti- tuierten Arylrest, eine Dialkylaminogruppe wobei jeder Alkylrest 1 bis 4 Kohlenstoffatome aufweisen kann, oder eine Nitrogruppe bedeuten können
einsetzt.

3. Verfahren nach Anspruch 1 oder 2, **dadurch gekennzeichnet, dass** man die Cyclisierung in Gegenwart einer organischen Säure durchführt.

4. Verfahren nach Anspruch 1 oder 2, **dadurch gekennzeichnet, dass** man die Cyclisierung in Gegenwart einer Carbonsäure mit 1 bis 20 Kohlenstoffatomen durchführt.

5. Verfahren nach Anspruch 4, **dadurch gekennzeichnet dass** man die Cyclisierung in Gegenwart von Ameisensäure, Essigsäure, Propionsäure, Buttersäure, Valeriansäure, Oxalsäure, Malonsäure, Bernsteinsäure und/oder Citronellsäure der Formel (V) 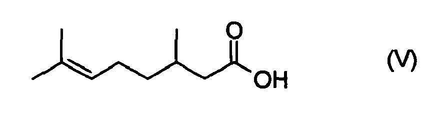 durchführt.

6. Verfahren nach Anspruch 1 oder 2, **dadurch gekennzeichnet, dass** man die Cyclisierung in Gegenwart einer anorganischen Säure durchführt.

7. Verfahren nach Anspruch 6, **dadurch gekennzeichnet, dass** man als anorganische Säure HCl, Schwefelsäure, Salpetersäure und/oder Phosphorsäure einsetzt.

8. Verfahren nach Anspruch 1 oder 2, **dadurch gekennzeichnet, dass** man die Cyclisierung in Gegenwart eines Carbonsäureanhydrids der Formel (VI) 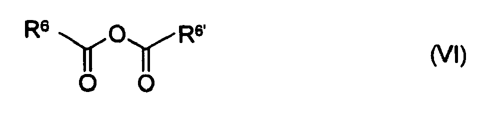 wobei
R⁶, R^{6'} gleich oder verschieden sind und einen verzweigten oder unver- zweigten C₁- bis C₁₂- Alkylrest oder C₇- bis C₁₂-Aralkylrest oder ei- nen C₆- bis C₁₀-Arylrest bedeuten, wobei die genannten Reste je- weils einen oder mehrere gleiche oder verschiedene Substituen- ten, ausgewählt aus der Gruppe OR⁷, SR⁸ NR⁹R¹⁰ und Halogen aufweisen können und wobei R⁶ und R^{6'} gemeinsam auch einen 5- bis 8-gliedrigen Ring bilden können, der eine oder mehrere ethy- lenische Doppelbindungen und ein oder mehrere gleiche oder ver- schiedene Heteroatome, ausgewählt aus der Gruppe O, S, NR¹¹ aufweisen kann und wobei R⁷, R⁸, R⁹, R¹⁰ und R¹¹ unabhängig voneinander C₁- bis C₆-Alkyl, C₇- bis C₁₂-Aralkyl und/oder substitu- iertes oder unsubstituiertes C₆- bis C₁₀-Aryl bedeuten können,
oder in Gegenwart eines Aldehyds der Formel (VII) 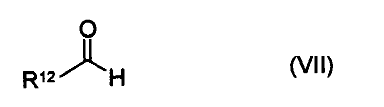 wobei
R¹² einen verzweigten oder unverzweigten C₁- bis C₁₂- Alkylrest oder C₇- bis C₁₂-Aralkylrest oder einen C₆- bis C₁₀-Arylrest bedeutet, wobei die genannten Reste jeweils einen oder mehrere gleiche oder verschiedene Substituenten, ausgewählt aus der Gruppe OR⁷, SR⁸ NR⁹R¹⁰ und Halogen aufweisen können, wobei R⁷, R⁸, R⁹ und R¹⁰ unabhängig voneienander die vorstehend genannten Bedeutungen haben können,
bedeutet,
oder in Gegenwart eines Ketons der Formel (VIII) 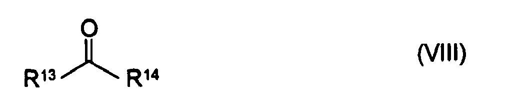 wobei
R¹³, R¹⁴ gleich oder verschieden sind und jeweils einen verzweigten o- der unverzweigten C₁- bis C₁₂- Alkylrest oder C₇- bis C₁₂- Aralkylrest oder einen C₆- bis C₁₀-Arylrest bedeuten, wobei die genannten Reste jeweils einen oder mehrere gleiche oder ver- schiedene Substituenten, ausgewählt aus der Gruppe OR⁷, SR⁸ NR⁹R¹⁰ und Halogen aufweisen können, und wobei R¹³ und R¹⁴ gemeinsam auch einen 5- bis 8-gliedrigen Ring bilden können, der eine oder mehrere ethylenische Doppelbindungen und ein oder mehrere gleiche oder verschiedene Heteroatome ausgewählt aus der Gruppe O, S, NR¹¹ aufweist und wobei R⁷, R⁸, R⁹, R¹⁰ und R¹¹ unabhängig voneinander die vorstehend angegebenen Bedeutungen haben können,
bedeuten,
oder in Gegenwart eines Vinylethers der allgemeinen Formel (IX) 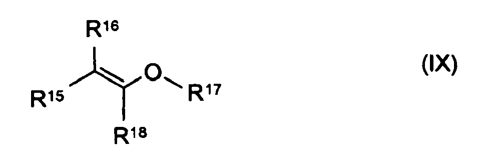 wobei
R¹⁵, R¹⁶, R¹⁷, R¹⁸ gleich oder verschieden sind und unabhängig voneinander je- weils einen verzweigten oder unverzweigten C₁- bis C₁₂- Alkyl- rest oder C₇- bis C₁₂-Aralkylrest oder einen C₆- bis C₁₀-Arylrest bedeuten, wobei die genannten Reste jeweils einen oder meh- rere gleiche oder verschiedene Substituenten, ausgewählt aus der Gruppe Oxo, OR⁷, SR⁸ NR⁹R¹⁰ und Halogen aufweisen können und wobei R¹⁶ und R¹⁷ gemeinsam auch einen 5- bis 8- gliedrigen Ring bilden können, der eine oder mehrere ethyleni- sche Doppelbindungen und ein oder mehrere gleiche oder ver- schiedene Heteroatome ausgewählt aus der Gruppe O, S, NR¹¹ aufweisen kann und wobei R⁷, R⁸, R⁹, R¹⁰ und R¹¹ unabhängig voneinander die vorstehend angegebenen Bedeutungen haben können,
durchführt.

9. Verfahren nach einem der Ansprüche 1, 2 oder 8, **dadurch gekennzeichnet, dass** man die Cyclisierung in Gegenwart von Essigsäureanhydrid, Propionsäureanhydrid, Pivalinsäureanhydrid und/oder Benzoesäureanhydrid durchführt.

10. Verfahren nach einem der Ansprüche 1, 2 oder 8, **dadurch gekennzeichnet, dass** man die Cyclisierung in Gegenwart von Acetaldehyd, Propionaldehyd, Chloral, 1,1,1-Trifluoraceton, 1,1,1-Trifluoracetophenon oder Hexafluoraceton durchführt.

11. Verfahren nach einem der Ansprüche 1 bis 10 zur Herstellung von optisch aktivem Isopulegol umfassend die Cyclisierung von optisch aktivem Citronellal.

12. Verfahren nach einem der Ansprüche 1 bis 11 zur Herstellung von L-Isopulegol der Formel (X) 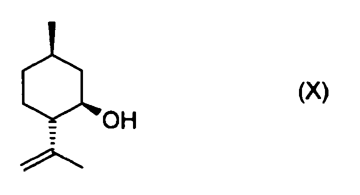 umfassend die Cyclisierung von D-Citronellal der Formel (XI)

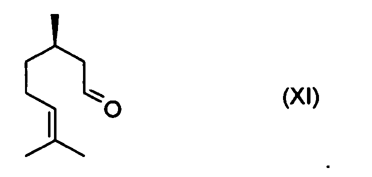

13. Verfahren zur Herstellung von racemischem oder optisch aktivem Menthol umfassend die Herstellung von racemischem oder optisch aktivem Isopulegol gemäß einem der Ansprüche 1 bis 12 und Hydrierung der ethylenischen Doppelbindung des so hergestellten Isopulegols.

## Claims

1. A process for the preparation of isopulegol of formula (I): 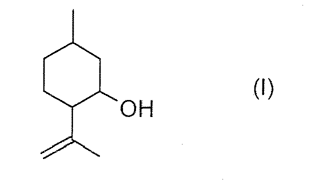 comprising the cyclization of citronellal of formula (II): 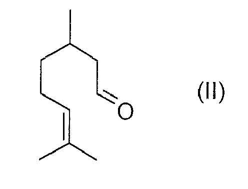 in the presence of a tris(aryloxy)aluminum catalyst of formula (III): 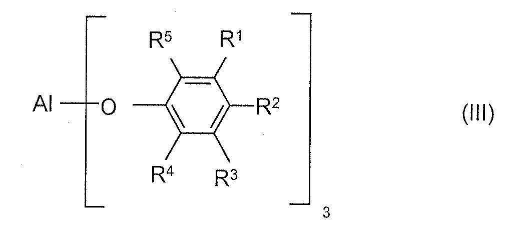 in which
Al is aluminum,
R¹, R², R³ independently of one another can each be hydrogen, a halogen atom, an alkyl radical having 1 to 8 carbon atoms, an alkoxy group having 1 to 8 carbon atoms, a substituted or unsubstituted aryl radical, a dialkylamino group in which each alkyl radical can have 1 to 4 carbon atoms, or a nitro group, and
R⁴, R⁵ independently of one another can each be a halogen atom, an alkyl radical having 1 to 8 carbon atoms, an alkoxy group having 1 to 8 carbon atoms, a dialkylamino group in which each alkyl radical can have 1 to 4 carbon atoms, a nitro group or a substituted or un- substituted aryl or heteroaryl radical,
wherein the cyclization is carried out in the presence of
I. at least one acid and/or
II. at least one compound selected from the group comprising carboxylic anhydrides, vinyl ethers, ketones and aldehydes of formula (VII): 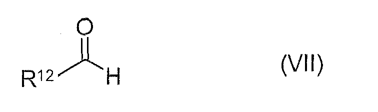 in which
R¹² is a branched or unbranched C₁- to C₁₂-alkyl radical or C₇- to C₁₂-aralkyl radical or a C₆- to C₁₀-aryl radical, it being possible for said radicals each to have one or more identical or different substituents selected from the group comprising OR⁷, SR⁸, NR⁹R¹⁰ and halogen, it being possible for R⁷, R⁸, R⁹ and R¹⁰ independently of one another to be C₁- to C₆-alkyl, C₇- to C₁₂-aralkyl and/or substituted or unsubstituted C₆- to C₁₀-aryl.

2. The process according to claim 1 wherein the tris(aryloxy)aluminum catalyst used is a tris(2,6-diarylaryloxy)aluminum catalyst of formula (IV): 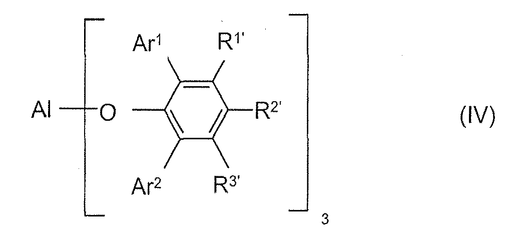 in which
Al is aluminum,
Ar¹, Ar² independently of one another are a substituted or unsubstituted aryl or heteroaryl radical, and
R^{1'}, R^{2'}, R^{3'} independently of one another can each be hydrogen, a halogen atom, an alkyl radical having 1 to 8 carbon atoms, an alkoxy group having 1 to 8 carbon atoms, a substituted or unsubstituted aryl radical, a dialkylamino group in which each alkyl radical can have 1 to 4 carbon atoms, or a nitro group.

3. The process according to claim 1 or 2 wherein the cyclization is carried out in the presence of an organic acid.

4. The process according to claim 1 or 2 wherein the cyclization is carried out in the presence of a carboxylic acid having 1 to 20 carbon atoms.

5. The process according to claim 4 wherein the cyclization is carried out in the presence of formic acid, acetic acid, propionic acid, butyric acid, valeric acid, oxalic acid, malonic acid, succinic acid and/or citronellic acid of formula (V):

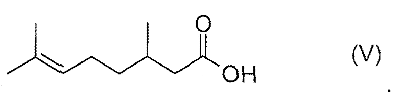

6. The process according to claim 1 or 2 wherein the cyclization is carried out in the presence of an inorganic acid.

7. The process according to claim 6 wherein the inorganic acid used is HCl, sulfuric acid, nitric acid and/or phosphoric acid.

8. The process according to claim 1 or 2 wherein the cyclization is carried out in the presence of a carboxylic anhydride of formula (VI): 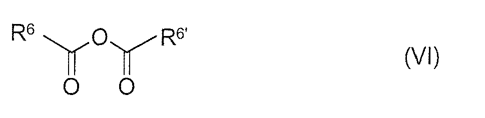 in which
R⁶, R^{6'} are identical or different and are a branched or unbranched C₁- to C₁₂-alkyl radical or C₇- to C₁₂-aralkyl radical or a C₆- to C₁₀-aryl radical, it being possible for said radicals each to have one or more identical or different substituents selected from the group comprising OR⁷, SR⁸, NR⁹R¹⁰ and halogen, and in which R⁶ and R^{6'} together can also form a 5- to 8-membered ring which can have one or more ethylenic double bonds and one or more identi- cal or different heteroatoms selected from the group comprising O, S and NR¹¹, it being possible for R⁷, R⁸, R⁹, R¹⁰ and R¹¹ independ- ently of one another to be C₁- to C₆-alkyl, C₇- to C₁₂-aralkyl and/or substituted or unsubstituted C₆- to C₁₀-aryl,
or in the presence of an aldehyde of formula (VII): 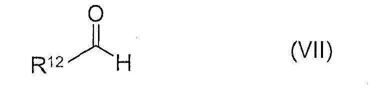 in which
R¹² is a branched or unbranched C₁- to C₁₂-alkyl radical or C₇- to C₁₂- aralkyl radical or a C₆- to C₁₀-aryl radical, it being possible for said radicals each to have one or more identical or different substitu- ents selected from the group comprising OR⁷, SR⁸, NR⁹R¹⁰ and halogen, it being possible for R⁷, R⁸, R⁹ and R¹⁰ independently of one another to be as defined above,
or in the presence of a ketone of formula (VIII): 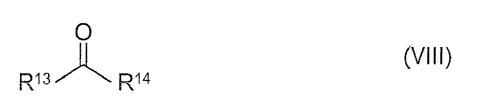 in which
R¹³, R¹⁴ are identical or different and are each a branched or un- branched C₁- to C₁₂-alkyl radical or C₇- to C₁₂-aralkyl radical or a C₆- to C₁₀-aryl radical, it being possible for said radicals each to have one or more identical or different substituents selected from the group comprising OR⁷, SR⁸, NR⁹R¹⁰ and halogen, and in which R¹³ and R¹⁴ together can also form a 5- to 8- membered ring which has one or more ethylenic double bonds and one or more identical or different heteroatoms selected from the group comprising O, S and NR¹¹, it being possible for R⁷, R⁸, R⁹, R¹⁰ and R¹¹ independently of one another to be as defined above,
or in the presence of a vinyl ether of general formula (IX): 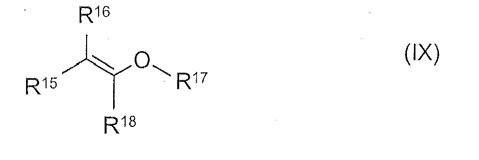 in which
R¹⁵, R¹⁶, R¹⁷, R¹⁸ are identical or different and independently of one another are each a branched or unbranched C₁- to C₁₂-alkyl radical or C₇- to C₁₂-aralkyl radical or a C₆- to C₁₀-aryl radical, it being possible for said radicals each to have one or more identical or different substituents selected from the group comprising oxo, OR⁷, SR⁸, NR⁹R¹⁰ and halogen, and in which R¹⁶ and R¹⁷ together can also form a 5- to 8-membered ring which can have one or more ethylenic double bonds and one or more identical or different heteroatoms selected from the group comprising O, S and NR¹¹, it being possible for R⁷, R⁸, R⁹, R¹⁰ and R¹¹ independently of one another to be as defined above.

9. The process according to one of claims 1, 2 and 8 wherein the cyclization is carried out in the presence of acetic anhydride, propionic anhydride, pivalic anhydride and/or benzoic anhydride.

10. The process according to one of claims 1, 2 and 8 wherein the cyclization is carried out in the presence of acetaldehyde, propionaldehyde, chloral, 1,1,1-trifluoroacetone, 1,1,1-trifluoroacetophenone or hexafluoroacetone.

11. The process according to one of claims 1 to 10 for the preparation of optically active isopulegol, comprising the cyclization of optically active citronellal.

12. The process according to one of claims 1 to 11 for the preparation of L-isopulegol of formula (X): 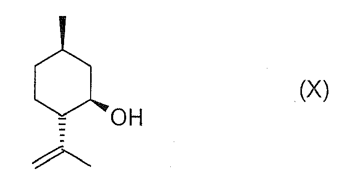 comprising the cyclization of D-citronellal of formula (XI):

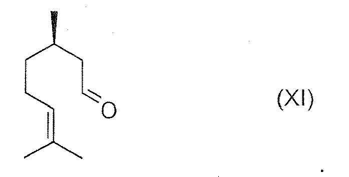

13. A process for the preparation of racemic or optically active menthol, comprising the preparation of racemic or optically active isopulegol according to one of claims 1 to 12, and hydrogenation of the ethylenic double bond of the isopulegol prepared in this way.

## Revendications

1. Procédé pour la préparation d'isopulegol de formule (I) 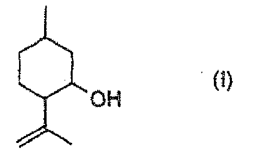 comprenant la cyclisation de citronellal de formule (II) 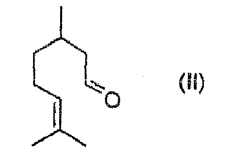 en présence d'un catalyseur de type tris(aryloxy)aluminium de formule (III) 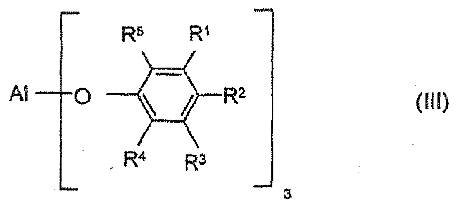 où
Al signifie aluminium et
R¹, R², R³ peuvent signifier à chaque fois, indépendamment l'un de l'autre, hydrogène, un atome d'halogène, un radical alkyle comprenant 1 à 8 atomes de carbone, un groupe alcoxy comprenant 1 à 8 atomes de carbone, un radical aryle substitué ou non substitué, un groupe dialkylamino, où chaque radical alkyle peut présenter 1 à 4 atomes de carbone, ou un groupe nitro et
R⁴, R⁵ peuvent signifier, à chaque fois indépendamment l'un de l'autre, un atome d'halogène, un radical alkyle comprenant 1 à 8 atomes de carbone, un groupe alcoxy comprenant 1 à 8 atomes de carbone, un groupe dialkylamino où chaque radical alkyle peut présenter 1 à 4 atomes de carbone, un groupe nitro ou un radical aryle ou hétéroaryle substitué ou non substitué,
**caractérisé en ce qu'**on réalise la cyclisation en présence
I. d'au moins un acide et/ou
II. d'au moins un composé choisi dans le groupe des anhydrides d'acide carboxylique, des vinyléthers, des cétones et des aldéhydes de formule (VII) 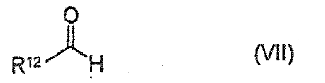 où
R¹² signifie un radical C₁-C₁₂-alkyle ramifié ou non ramifié ou un radical C₇-C₁₂-aralkyle ou un radical C₆-C₁₀-aryle, où les radicaux mentionnés peuvent présenter à chaque fois un ou plusieurs substituants identiques ou différents, choisis dans le groupe OR⁷, SR⁸, NR⁹R¹⁰ et halogène, où R⁷, R⁸, R⁹ et R¹⁰ peuvent représenter, indépendamment l'un de l'autre, C₁-C₆-alkyle, C₇-C₁₂-aralkyle et/ou C₆-C₁₀-aryle substitué ou non substitué.

2. Procédé selon la revendication 1, **caractérisé en ce qu'**on utilise comme catalyseur de type tris(aryloxy)aluminium un catalyseur de type tris(2,6-diarylaryloxy)aluminium de formule (IV) 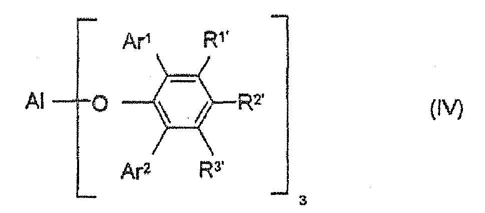 où
Al signifie aluminium et
Ar¹, Ar² représentent à chaque fois indépendamment l'un de l'autre un radical aryle ou hétéroaryle substitué ou non substitué et
R^{1'}, R^{2'}, R^{3'} peuvent signifier à chaque fois, indépendamment l'un de l'autre, hydrogène, un atome d'halogène, un radical alkyle comprenant 1 à 8 atomes de carbone, un groupe alcoxy comprenant 1 à 8 atomes de carbone, un radical aryle substitué ou non substitué, un groupe dialkylamino, où chaque radical alkyle peut présenter 1 à 4 atomes de carbone, ou un groupe nitro.

3. Procédé selon la revendication 1 ou 2, **caractérisé en ce qu'**on réalise la cyclisation en présence d'un acide organique.

4. Procédé selon la revendication 1 ou 2, **caractérisé en ce qu'**on réalise la cyclisation en présence d'un acide carboxylique comprenant 1 à 20 atomes de carbone.

5. Procédé selon la revendication 4, **caractérisé en ce qu'**on réalise la cyclisation en présence d'acide formique, acétique, propionique, butyrique, valérianique, oxalique, malonique, succinique et/ou citronellique de formule (V)

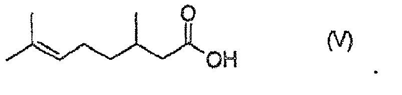

6. Procédé selon la revendication 1 ou 2, **caractérisé en ce qu'**on réalise la cyclisation en présence d'un acide inorganique.

7. Procédé selon la revendication 6, **caractérisé en ce qu'**on utilise comme acide inorganique, HCl, de l'acide sulfurique, de l'acide nitrique et/ou de l'acide phosphorique.

8. Procédé selon la revendication 1 ou 2, **caractérisé en ce qu'**on réalise la cyclisation en présence d'un anhydride d'acide carboxylique de formule (VI) 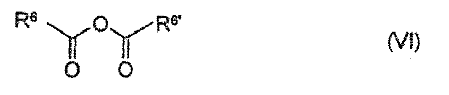 où
R⁶, R⁶ ^{'} sont identiques ou différents et signifient un radical C₁-C₁₂-alkyle ramifié ou non ramifié ou un radical C₇-C₁₂-aralkyle ou un radical C₆-C₁₀-aryle, où les radicaux mentionnés peuvent présenter à chaque fois un ou plusieurs substituants identiques ou différents, choisis dans le groupe formé par OR⁷, SR⁸, NR⁹R¹⁰ et halogène et où R⁶ et R^{6'} peuvent également former, ensemble, un cycle de 5 à 8 chaînons, qui peut présenter une ou plusieurs doubles liaisons éthyléniques et un ou plusieurs hétéroatomes identiques ou différents, choisis dans le groupe formé par O, S, NR¹¹ et où R⁷, R⁸, R⁹, R¹⁰ et R¹¹ peuvent signifier, indépendamment l'un de l'autre, C₁-C₆-alkyle, C₇- C₁₂-aralkyle et/ou C₆-C₁₀-aryle substitué ou non substitué,
ou en présence d'un aldéhyde de formule (VII) 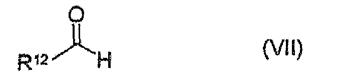 où
R¹² signifie un radical C₁-C₁₂-alkyle ramifié ou non ramifié ou un radical C₇-C₁₂-aralkyle ou un radical C₆-C₁₀-aryle, où les radicaux mentionnés peuvent présenter à chaque fois un ou plusieurs substituants identiques ou différents, choisis dans le groupe OR⁷, SR⁸, NR⁹R¹⁰ et halogène, où R⁷, R⁸, R⁹ et R¹⁰ peuvent présenter, indépendamment l'un de l'autre, les significations susmentionnées,
ou en présence d'une cétone de formule (VIII) 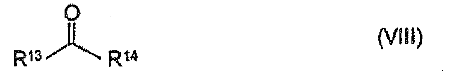 où
R¹³, R¹⁴ sont identiques ou différents et signifient un radical C₁-C₁₂-alkyle ramifié ou non ramifié ou un radical C₇-C₁₂-aralkyle ou un radical C₆-C₁₀-aryle, où les radicaux mentionnés peuvent présenter à chaque fois un ou plusieurs substituants identiques ou différents, choisis dans le groupe formé par OR⁷, SR⁸, NR⁹R¹⁰ et halogène et où R¹³ et R¹⁴ peuvent également former, ensemble, un cycle de 5 à 8 chaînons, qui peut présenter une ou plusieurs doubles liaisons éthyléniques et un ou plusieurs hétéroatomes identiques ou différents, choisis dans le groupe formé par O, S, NR¹¹ et où R⁷, R⁸, R⁹, R¹⁰ et R¹¹ peuvent présenter, indépendamment l'un de l'autre, les significations susmentionnées
ou en présence d'un vinyléther de formule générale (IX) 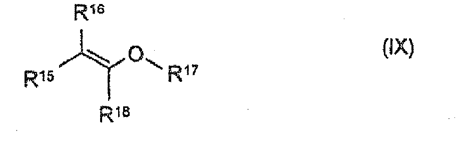 où
R¹⁵, R¹⁶, R¹⁷, R¹⁸ sont identiques ou différents et signifient indépendamment l'un de l'autre, à chaque fois un radical C₁-C₁₂-alkyle ramifié ou non ramifié ou un radical C₇-C₁₂-aralkyle ou un radical C₆-C₁₀-aryle, où les radicaux mentionnés peuvent présenter à chaque fois un ou plusieurs substituants identiques ou différents, choisis dans le groupe formé par oxo, OR⁷, SR⁸, NR⁹R¹⁰ et halogène et où R¹⁶ et R¹⁷ peuvent également former, ensemble, un cycle de 5 à 8 chaînons, qui peut présenter une ou plusieurs doubles liaisons éthyléniques et un ou plusieurs hétéroatomes identiques ou différents, choisis dans le groupe formé par O, S, NR¹¹ et où R⁷, R⁸, R⁹, R¹⁰ et R¹¹ peuvent présenter, indépendamment l'un de l'autre, les significations susmentionnées.

9. Procédé selon l'une quelconque des revendications 1, 2 ou 8, **caractérisé en ce qu'**on réalise la cyclisation en présence d'anhydride de l'acide acétique, d'anhydride de l'acide propionique, d'anhydride de l'acide pivalique et/ou d'anhydride de l'acide benzoïque.

10. Procédé selon l'une quelconque des revendications 1, 2 ou 8, **caractérisé en ce qu'**on réalise la cyclisation en présence acétaldéhyde, de propionaldéhyde, de chloral, de 1,1,1-trifluoroacétone, de 1,1,1-trifluoroacétophénone ou d'hexafluoroacétone.

11. Procédé selon l'une quelconque des revendications 1 à 10 pour la préparation d'isopulegol optiquement actif comprenant la cyclisation de citronellal optiquement actif.

12. Procédé selon l'une quelconque des revendications 1 à 11 pour la préparation de L-isopulegol de formule (X) 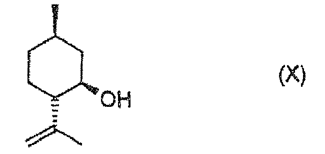 comprenant la cyclisation de D-citronellal de formule (XI)

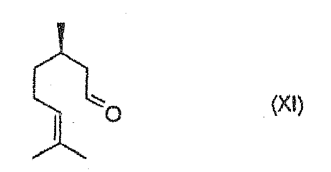

13. Procédé pour la préparation de menthol racémique ou optiquement actif comprenant la préparation d'isopulegol racémique ou optiquement actif selon l'une quelconque des revendications 1 à 12 et l'hydrogénation de la double liaison éthylénique de l'isopulegol ainsi préparé.
